# EUROPÄISCHE PATENTSCHRIFT

(11) **EP 1 047 459 B1**
(45) Veröffentlichungstag und Bekanntmachung des Hinweises auf die Patenterteilung: **18.09.2002**
(21) Anmeldenummer: 98958226.7
(22) Anmeldetag: 19.10.1998
(51) Int. Cl.: A61L 27/00

(54) **IMPLANTAT AUS RESORBIERBAREN PHOSPHATGLAS**
IMPLANT MADE OF RESORBABLE PHOSPHATEGLASS
IMPLANT CONSTITUE DE VERRE PHOSPHATE RESORBABLE

(30) Priorität: 21.10.1997 DE 19746464; 16.01.1998 DE 19801428; 05.02.1998 DE 19804520; 14.08.1998 DE 19836985
(43) Veröffentlichungstag der Anmeldung: 02.11.2000
(73) Patentinhaber: augmentec Aktiengesellschaft Implantologie-Epithetik, 91799 Langenaltheim (DE)
(72) Erfinder: GEHL, Gerolf, CH-8700 Küsnacht (CH)
(74) Vertreter: Thiel, Christian, Dr. Dipl.-Chem.
(86) Internationale Anmeldenummer: EP9806599
(87) Internationale Veröffentlichungsnummer: WO99020319

(56) Entgegenhaltungen:
- WO-A-93/15721
- WO-A-94/04657
- WO-A-96/39202
- US-A- 5 149 368
- US-A- 5 306 303
- US-A- 5 324 294
- US-A- 5 462 722
- US-A- 5 571 182

## Beschreibung

Die Erfindung betrifft ein poröses Implantat, das insbesondere als Brustimplantat verwandt werden kann.

In der Chirurgie sind Implantate wie Epithesen zum Ausgleich von angeborenen oder erworbenen Körperdefekten seit langem bekannt und vielfach beschrieben. Epithesen sind individuell modellierte Organersatzstücke aus Werkstoffen, die insbesondere zur Verdeckung von Gesichtsdefekten einschließlich der Ohren, aber auch zur Anwendung unter funktionellen Gesichtspunkten in der Prothetik eingesetzt werden. Implantate sind natürliche oder künstliche, in der Regel dauerhafte Formteile, die als plastischer Ersatz oder zur mechanischen Verstärkung in den Körper eingebracht werden, z. B. Augenbulbus-Epithesen.

Implantate und Epithesen werden vielfach eingesetzt, um die Folgen von Unfällen oder chirurgischen Eingriffen zu verdecken und eine natürliche Körpererscheinung vorzuspiegeln. Ein häufiger Einsatzzweck ist die Nachbildung oder Vergrößerung der weiblichen Brust, beispielsweise zum Ausgleich natürlicher Defekte oder Unzulänglichkeiten oder nach deren chirurgischer Entfernung, bedingt durch bösartige Tumore. Weitere Einsatzgebiete sind der Ausgleich angeborener, verletzungs- oder operationsbedingter. Defekte im Kopfbereich.

Bekannt geworden sind in der ästhetischen Brustchirurgie insbesondere Implantate in Form von silikongefüllten Kissen. Implantate zum Ersatz oder zur Stabilisierung von Knochen oder Knochenteilen werden vielfach eingesetzt.

Im Bereich der Übergangsformen von Epithesen zu Implantaten geht es vielfach um die Abdeckung einer defekten Körperoberfläche gegen unerwünschte äußere Einflüsse, also auch beispielsweise zur Unterstützung der Wundheilung und zur Verbesserung der Hygiene. In diesem Bereich stellen Epithesen aber eine nicht immer optimale Lösung der bestehenden Probleme dar. Ein grundsätzlicher Nachteil von Epithesen ist, daß sie nur geeignet sind, ein Körperdefekt optisch auszugleichen, immer jedoch einen Fremdkörper darstellen.

Unter diesen Gesichtspunkten wären Lösungen wünschenswert, die geeignet sind, die nach einer Operation oder Verwundung zurückbleibende Wunde zu schließen oder Körperhöhlung auszufüllen, und die gleichzeitig die Invasion von körpereigenem Gewebe ermöglichen. Auf diese Art und Weise könnte eine vollständige Integration eines Implantats in den Körper erreicht werden. Im Idealfall wäre so eine Neuformung der Körperkonturen über eine Modellform möglich.

Ferner wäre es wünschenswert, über Implantate zu verfügen, die so gestaltet, behandelt und strukturiert sind, daß sie das Einwachsen von körpereigenem Gewebe in das Innere fördern. Insbesondere im Bereich der ästhetischen Brustchirurgie wäre es ferner wünschenswert, wenn ein die Brust nachbildendes Implantat während und/oder nach der Invasion körpereigenen Gewebes vom Körper langsam resorbiert wird, so daß sein Platz vollständig von Körpergewebe eingenommen wird.

Der Erfindung liegt die Aufgabe zugrunde, ein Implantat bereitzustellen, das die oben geschilderten Nachteile herkömmlicher Epithesen und Implantate vermeidet, dennoch in medizinischer Hinsicht unbedenklich und zudem gewebefreundlich ist. Das Implantat soll über seine Funktionsdauer formstabil bleiben und so strukturiert sein, daß eine Invasion körpereigenen Gewebes stattfinden kann und eine zumindest teilweise Resorption möglich ist.

Diese Aufgabe wird mit einem porösen Implantat gelöst, das aus einem körperverträglichen, vom Körper resorbierbaren Phosphatglas besteht.

Das erfindungsgemäße Implantat besteht aus einem körperverträglichen Phosphatglas. Körperverträglich bedeutet insbesondere auch gewebeverträglich in dem Sinne, daß eine Invasion von körpereigenem Gewebe in das Implantat stattfinden kann. Das Material soll vor allem das Einbluten bzw. die Ausbildung von Blutgefäßen fördern, eine der Voraussetzungen für die Resorbierbarkeit durch den Körper sowie für die Invasion körpereigenen Gewebes.

Unter Porenstruktur wird eine jede schwamm-, schaum- oder wabenartige Struktur verstanden, d. h. eine jede Struktur, die mit unregelmäßig oder regelmäßig geformten Hohlräumen ausgestattet ist. Auf diese Art und Weise wird nicht nur das Gewicht des Implantats niedrig gehalten, sondern auch das Einbluten und die Invasion von körpereigenem Gewebe erleichtert. Das Material des Implantats kann, je nach gewünschtem Zweck, aus mehr oder weniger festen Materialien bestehen. So ist es beispielsweise zur Nachbildung von Nasen, Augen, Ohren und Ohrenteilen zweckmäßig, ein festeres Material einzusetzen. Je nach Einsatzzweck kann der Einsatz eines resorptionsbeständigeren Materials oder eines im Körper schneller resorbierbaren Materials zweckmäßig sein. Insbesondere bei Brustimplantaten ist es vorteilhaft, ein im Körper resorbierbares Material einzusetzen, so daß eine allmähliche vollständige Verdrängung stattfinden kann.

Bevorzugtes Material für das erfindungsgemäße Implantat ist Glaskeramik.

Die Materialien haben den Vorteil, daß sie in der Anfangsphase, d. h. nach der Implantation, ein festes porenförmiges Stützskelett ausbildet, das über längere Zeit stabil bleibt. Nach erfolgter Invasion des körpereigenen Gewebes und Ein- und Durchblutung kommt es zu einer beschleunigten Auflösung bzw. Resorption von innen heraus, die das Stützskelett beseitigt und Raum für die Ausdehnung der körpereigenen Zellen schafft.

Glaskeramik weist den Vorteil der Fräsbarkeit auf und ist als Knochenersatzstoff bereits medizinisch erprobt. Materialien mit mehr oder weniger großer Biolöslichkeit sind bekannt. Glaskeramiken sind voll körperverträglich und werden, je nach Einstellung, im Verlauf von einigen Monaten bis zu mehreren Jahren, vollständig resorbiert. Solche Materialien sind beispielsweise in J. Vogel et al;, Bioceramics, 1997, 57; Angew. Chem. Int. Ed. Engl. 26 (1987), 527; und Glastech. Bir.Glassci.technol. 70 (1997), 220 beschrieben. Spongiöse Glaskeramik wird beispielsweise von der Firma Biovision in Ilmenau, Deutschland unter der Bezeichnung "Bioverit" vertrieben. Siehe auch DE 196 14 421 A1, die einen Polymer-gebundenen Knochenersatzstoff betrifft.

Bei der Gestaltung der erfindungsgemäßen Implantate für die Anwendung insbesondere im Brustbereich ist es vorteilhaft, einen Aufbau vorzusehen, bei dem das in etwa halbkugelförmige Implantat aus einem leicht resorbierbaren Kern und einer langsam resorbierbaren äußeren Halbschale besteht. Der Kern liegt damit körperseitig frei und wird somit schneller resorbiert als die schützende und stützende Halbschale.

Um das Einwachsen von körpereigenem Gewebe zu fördern, ist es zweckmäßig, das Implantat mit einer Hülle oder Beschichtung zu versehen, die das Einwachsen des Implantats in den Körper und die Ausbildung von Gewebe im Implantat fördert. Eine solche Hülle oder Beschichtung kann vorteilhaft aus Keratinocyten bestehen, wie sie an und für sich für die Behandlung und den Verschluß von Brandwunden bekannt sind. Es können aber auch Collagen oder Stoffe verwandt werden, die die Collagenbindung an Körperzellen fördern. In Frage kommen hier insbesondere natürliche oder synthetische Eiweißstoffe, wie sie beispielsweise in WO-A-91/02437, WO-A-93/11781 und von J.J. Qian und R.S. Bahtnagar in J. Biomed. Mater. Res. 31, 545 (1996) beschrieben sind.

Wie bereits beschrieben, ist Glaskeramik das erfindungsgemäß besonders bevorzugte resorbierbare Material. Dieses Material hat eine Porenstruktur und liegt zweckmäßigerweise in Form eines durch Fräsen und/oder Formgießen vormodellierten Formkörpers vor.

Erfindungsgemäße Implantate aus Glaskeramik werden auf an und für sich bekannte Art aus Phosphatglasgranulat in einer Form gesintert. Die Sintertemperatur beträgt im allgemeinen 600 bis 800°C, was zu einer oberflächlichen Verschmelzung und Versinterung des Granulats führt. Gleichzeitig tritt bei diesem Sinterungsvorgang eine teilweise Kristallisation auf. Die Korngröße des Granulats liegt zweckmäßigerweise im Bereich von 200 bis 500 µm. Die chemische Zusammensetzung eines für diese Zwecke verwandten Phosphatglases beträgt beispielsweise 32,6 mol-% P₂O₅, 27,6 mol-% CaO, 27,6 mol-% Na₂O und 12,2 mol-% MgO. Der Na₂O-Gehalt kann ganz oder teilweise durch K₂O ausgetauscht werden. Solche Materialien können nach dem Sintern beispielsweise einen offenen Porenanteil von 65% aufweisen, wobei die Porendurchmesser zum überwiegenden Teil im Bereich von 150µm bis 400µm liegen.

Das Phosphatglas bzw. die Implantate können durch geeignete Dotierung mit Alkali-Ionen auf einen gewünschten pH-Wert eingestellt werden. Dieser kann zum einen im physiologischen Bereich liegen, zum anderen aber auch im basischen Bereich, beispielsweise zwischen pH 7,7 und 8,7, vorzugsweise im Bereich von pH 7,9 bis pH 8,4. Es ist bekannt, daß Tumore das normalerweise neutrale bis schwach basische Milieu des Zellgewebes in den sauren Bereich hinein verschieben und damit ein das Tumorwachstum unterstützendes Milieu schaffen. Ein Implantat, das mit die Basizität erhöhenden Ionen geladen ist, insbesondere Alkali- und Erdalkali-Ionen, vermag dieser Verschiebung in den sauren Bereich hinein entgegenzuwirken. Nach tumorbedingter chirurgischer Entfernung von Gewebeteilen kann eine basische Einstellung des zur Korrektur verwandten erfindungsgemäßen Implantats angezeigt sein.

Die erfindungsgemäßen Implantate können ergänzend mit Arzneimitteln beladen sein, insbesondere auch mit Antibiotika, Cytostatika und dergleichen, wobei insbesondere eine Einbettung in Frage kommt, die eine kontinuierliche oder schubweise Freisetzung im Rahmen der Resorption des Implantats gewährleistet. Eine Verwendung der Implantate als reiner Arzneimittelträger zur Behandlung von Krankheitszuständen durch sukzessive Abgabe des oder der Wirkstoffe kommt ebenfalls in Frage, wobei die Resorbierbarkeit des Implantats bevorzugt, aber nicht in jedem Fall erforderlich ist. Letzteres setzt die anschließende Explantation voraus.

Die erfindungsgemäßen Implantate sind insbesondere als Wundverschluß geeignet, d. h. zum direkten Auf- oder Einbringen in die offene Wunde, was das Einwachsen von Gewebe ermöglicht und fördert. Alle erfindungsgemäßen Implantate erlauben eine sonst nicht erreichbare Volumengewinnung. Mineralische Hartimplantate aus beispielsweise Glaskeramik oder Hydroxylapatit bieten darüber hinaus auch eine Stützfunktion, solange die Materialien noch nicht weitgehend resorbiert sind.

Bei Anwendungen im ästhetischen und kosmetischen Bereich etwa zur Vergrößerung der Brust, ergibt sich bei der Verwendung insbesondere von Glaskeramik der Vorteil, daß Verwachsungen des Narbengewebes weitgehend vermieden werden und es nicht zu Gewebeveränderungen kommt.

Es kann insbesondere zweckmäßig sein, die vormodellierten Implantate nicht nur mit einer die Zelleinwanderung fördernden Beschichtung oder Imprägnierung zu versehen, sondern ergänzend oder alternativ Zellen in das Implantat einzubringen und die eingebrachten Zellen in einer üblichen Nährlösung zu kultivieren. Das Einbringen von Zellen kann beispielsweise auch dadurch erfolgen, daß eine Nährlösung mit Zellen versetzt und das Implantat in diese Nährlösung eingelegt wird, wobei durch die Kapillarwirkung Zellen in die Hohlräume eingetragen werden. Bei den Zellen kann es sich um Fremdzellen handeln, die mit dem Gewebe des Empfängers des Implantats verträglich sind, oder aber auch um Zellen des Implantatempfängers, im Falle eines Brustimplantats beispielsweise um Zellen aus der erhaltenen Brust. Bevorzugt sind Fettzellen, denen ggf. zur Stabilisierung Antioxidantien beigegeben werden, oder auch humane Knorpelzellen. Zellmaterialien können beispielsweise von den Firmen Geistlich in Luzern, Schweiz und Maerz, Deutschland bezogen werden. Die Zellen werden zweckmäßigerweise für den speziellen Zweck angezüchtet und in der Nährlösung kultiviert.

In der Praxis wird so vorgegangen, daß im Falle einer chirurgischen Teilexzision einer weiblichen Brust wegen eines Tumors das vormodellierte, beschichtete und ggf. mit Zellen beaufschlagte Implantat unmittelbar in die Wunde eingebracht wird und die Wunde anschließend wieder verschlossen wird. Um die Blutversorgung des Implantats sicherzustellen bzw. zu fördern, kann eine Anastomose zwischen der verbliebenen, gesunden Brust und der implantierten Brust gelegt werden. Die Anastomose verbessert nicht nur die Einblutung, sondern fördert auch die Ernährung eingelagerter körpereigener Fettzellen aus der gesunden Brust und damit die Invasion von Körpergewebe in das Implantat.

Die erfindungsgemäßen Implantate sind auch besonders gut für den Einsatz bei kosmetischen Operationen der weiblichen Brust geeignet. Im Rahmen von Maßnahmen zur Vergrößerung der Brust aus kosmetischen Gründen, aber auch beispielsweise nach Geschlechtsumwandlungen können die erfindungsgemäßen Implantate mit Vorteil eingebracht werden. In der Regel wird dabei so vorgegangen, daß zunächst durch einen sogenannten Tissue-Expander in bekannter Weise die Haut nach Bedarf aufgedehnt wird. Nach Abschluß der Expansion wird die eigentliche Operation vorgenommen, mit der das Implantat eingebracht und in der Brust fixiert wird. Die Implantate sorgen zunächst für die richtige Formgebung und die Stabilisierung der vorgegebenen Form. In dem Maße, wie sich das Implantat auflöst und durch Gewebe ersetzt wird, entfällt die Stützfunktion und wird von dem neu gebildeten Gewebe übernommen.

Die Rekonstruktion ausgedehnter Gesichtsdefekte bei Knochen- und Weichteilverlust wird mit Keramikstrukturen unterschiedlicher Dichte und Resorptionszeit ermöglicht. Beispielsweise kann in einen großen Gesichtsdefekt (z. B. Operationshöhle nach Tumorentfernung) ein vorgeformter Implantatblock eingesetzt werden, der in sich bereits die Determinierung von Knochenersatz und Weichteilersatz enthält. Derartige Implantatblöcke lassen sich künstlerisch oder computergesteuert (stereolithographisch) aufbauen, so daß die Strukturen, die verknöchern sollen und die Vorstufe des Weichgewebes bereits durch spezielle determinierende Beladung im Implantatmaterial gespeichert sind.

Auf gleiche Weise können Knochen- und Weichteildefekte im Beinbereich oder in anderen Körperregionen einzeitig therapiert werden.

Das Implantatmaterial kann über seine Zusammensetzung, Dichte, Porosität und Gestaltung der jeweils gewünschten Resorptionszeit angepaßt werden.

Vergleichbar im Verfahren kann das erfindungsgemäße Implantat auch als Organersatz (beispielsweise Leber, Pankreas) eingesetzt werden. Nach Teilresektion beispielsweise der Leber, wird ein großporiges Keramikimplantat als Platzhalter eingebracht, das mit leberfunktionsspezifischen Zellen beladen oder das entsprechend mit Peptidverbindungen oder Wachstumsinduktoren imprägniert bzw. kodiert ist. Das Hartimplantat besteht zweckmäßigerweise aus einem leichter resorbierbaren Kern und einer schwerer resorbierbaren Hülle, die erst nach Auflösung des Kerns vollständig resorbiert wird. So ist gewährleistet, daß der Platz bis zur vollständigen Ausbreitung der funktionsspezifischen Zellen zur Verfügung steht. Anschließend, nach weitgehender Besiedelung und Ausfüllung mit den Organzellen (etwa 3 bis 4 Monate) findet die vollständige Resorption der Stützstrukturen statt, so daß im Endzustand weiches, funktionsspezifisches Gewebe den Platz einnimmt. Entsprechend läßt sich auch das gesamte Organ in mehreren Schritten austauschen, wozu mehrere Operations- und Einheilungsphasen entsprechender Segmente notwendig sind.

Die erfindungsgemäßen Implantate können zur Korrektur des Erscheinungsbildes insbesondere der weiblichen Brust eingesetzt werden, sowohl nach krankheitsbedingter chirurgischer Ausräumung von Gewebe, als auch nach Unfällen und damit verbundenen Gewebeverlust zur besseren Konturierung z. B. der Beine oder des Gesäßes oder auch zur Konturierung nach Verlust von Wangenteilen, Nasen-, Augen-, oder Ohrverlust, bzw. wegen Nichtanlage von Geburt.

## Patentansprüche

1. Poröses Implantat, insbesondere Brustimplantat, **dadurch gekennzeichnet, daß** es aus vollständig resorbierbarem, körperverträglichem Phosphatglas besteht.

2. Poröses Implantat nach Anspruch 1, **dadurch gekennzeichnet, daß** es durch Sintern von Phosphatglasgranulat in einer Korngröße von 200 bis 500µm erhalten wird.

3. Poröses Implantat nach einem der vorstehenden Ansprüche, **dadurch gekennzeichnet, daß** es eine Beschichtung aus Keratinocyten, Collagen oder einem Stoff, der die Collagenbindung an Körperzellen fördert, aufweist.

4. Poröses Implantat nach einem der vorstehenden Ansprüche, **dadurch gekennzeichnet, daß** die Beschichtung ein natürliches oder synthetisches Peptid ist.

5. Poröses Implantat nach einem der vorstehenden Ansprüche, **dadurch gekennzeichnet, daß** es aus Phosphatglas besteht, und auf einen pH-Wert von 7,7 bis 8,7 eingestellt ist.

6. Poröses Implantat nach Anspruch 5, **dadurch gekennzeichnet, daß** es vormodelliert ist und durch abtragende Bearbeitung korrigierbar und anpaßbar ist.

7. Poröses Implantat nach Anspruch 6, **dadurch gekennzeichnet, daß** es aus Phosphatglas besteht, die einen resorbierbaren Kern aufweist, der halbschalig mit einer resorbierenden Hülle versehen ist, worin der Kern zeitlich vor der halbschaligen Hülle resorbiert wird.

8. Poröses Implantat nach einem der Ansprüche 6 oder 7, **dadurch gekennzeichnet, daß** es mit lebensfähigen Zellen imprägniert ist.

9. Poröses Implantat nach Anspruch 8, **dadurch gekennzeichnet**, da es in einer zellhaltigen Nährlösung vorkultiviert ist.

10. Verwendung von vollständig resorbierbarem, körperverträglichem Phosphatglas nach einem der Ansprüche 1 bis 9 zur Herstellung von Implantaten zur Ergänzung und/oder Wiederherstellung von menschlichem Gewebe.

11. Verwendung von vollständig resorbierbarem, körperverträglichem Phosphatglas nach Anspruch 10 zur Herstellung von Implantaten bei der Wiederherstellung oder Vergrößerung der Brust.

## Claims

1. Porous implant, particularly a breast implant, **characterised in that** it comprises completely resorbable, body-compatible phosphate glass.

2. Porous implant as claimed in Claim 1, **characterised in that** it is obtained by sintering granular phosphate glass with a grain size of 200 to 500 µm.

3. Porous implant as claimed in one of the preceding claims, **characterised in that** it has a coating of keratinocytes, collagen or a material which promotes collagen binding to body cells.

4. Porous implant as claimed in one of the preceding claims, **characterised in that** the coating is a natural or synthetic peptide.

5. Porous implant as claimed in one of the preceding claims, **characterised in that** it comprises phosphate glass and is adjusted to a pH value of 7.7 to 8.7.

6. Porous implant as claimed in Claim 5, **characterised in that** it is pre-modelled and is correctable and adjustable by material-removing treatment.

7. Porous implant as claimed in Claim 6, **characterised in that** it comprises phosphate glass, which has a resorbable core, which is provided with a half shell of a resorbing sheath, wherein the core is resorbed before the half shell sheath.

8. Porous implant as claimed in one of Claims 6 or 7, **characterised in that** it is impregnated with viable cells.

9. Porous implant as claimed in Claim 8, **characterised in that** it is pre-cultivated in a cell-containing nutrient solution.

10. Use of completely resorbable, body-compatible phosphate glass as claimed in one of Claims 1 to 9 for producing implants to supplement and/or reconstruct human tissue.

11. Use of completely resorbable, body-compatible phosphate glass as claimed in Claim 10 for producing implants in the reconstruction or enlargement of the breast.

## Revendications

1. Implant poreux, en particulier implant mammaire, **caractérisé en ce qu'**il consiste en du verre phosphaté totalement résorbable, toléré par le corps.

2. Implant poreux selon la revendication 1 ou 2, **caractérisé en ce qu'**il est obtenu par frittage de granulés de verre phosphaté ayant une taille de grains de 200 à 500 µm.

3. Implant poreux selon l'une des revendications précédentes, **caractérisé en ce qu'**il présente un revêtement de kératinocytes, de collagène ou d'une substance qui favorise la formation de collagène sur les cellules corporelles.

4. Implant poreux selon l'une des revendications précédentes, **caractérisé en ce que** le revêtement est un peptide naturel ou synthétique.

5. Implant poreux selon l'une des revendications précédentes, **caractérisé en ce qu'**il consiste en du verre phosphaté et est ajusté à une valeur de pH de 7,7 à 8,7.

6. Implant poreux selon la revendication 5, **caractérisé en ce qu'**il est pré-modelé et peut être corrigé et ajusté par façonnage avec enlèvement.

7. Implant poreux selon la revendication 6, **caractérisé en ce qu'**il consiste en du verre phosphaté qui présente un noyau résorbable, qui est muni sur une demi-coque d'une enveloppe se résorbant, tandis que le noyau est résorbé dans le temps avant l'enveloppe en demi-coque.

8. Implant poreux selon l'une des revendications 6 ou 7, **caractérisé en ce qu'**il est imprégné avec des cellules viables.

9. Implant poreux selon la revendication 8, **caractérisé en ce qu'**il est pré-cultivé dans une solution nutritive contenant des cellules.

10. Utilisation de verre phosphaté totalement résorbable, toléré par le corps, selon l'une des revendications 1 à 9 pour la préparation d'implants pour compléter et/ou restaurer des tissus humains.

11. Utilisation de verre phosphaté totalement résorbable, toléré par le corps, selon la revendication 10 pour la préparation d'implants dans la restauration ou le développement de la poitrine.
